# EUROPEAN PATENT APPLICATION

(11) **EP 4 276 111 A1**
(43) Date of publication of application: **15.11.2023**
(21) Application number: 22736575.6
(22) Date of filing: 07.01.2022
(51) Int. Cl.: C07K 16/28, A61P 35/00, A61K 39/395, C12N 15/13

(54) **ANTIBODY SPECIFICALLY BINDING TO 4-1BB AND ANTIGEN-BINDING FRAGMENT OF ANTIBODY**

(30) Priority: 08.01.2021 CN 202110025248
(71) Applicant: Beijing Hanmi Pharmaceutical Co., Ltd., Beijing 101312 (CN)
(72) Inventor: LIU, Yang, Beijing 101312 (CN); ZHANG, Ruijing, Beijing 101312 (CN); FAN, Fei, Beijing 101312 (CN); LI, Xiaochun, Beijing 101312 (CN); YU, Lu, Beijing 101312 (CN); LIU, Jiawang, Beijing 101312 (CN); LEE, Kyoung Woo, Beijing 101312 (CN)
(74) Representative: Høiberg P/S
(86) International application number: PCT/CN2022/070627
(87) International publication number: WO 2022/148413

(57) **Abstract**

Provided are an antibody specifically binding to 4-1BB and an antigen-binding fragment thereof. The antibody specifically binding to 4-1BB and the antigen-binding fragment of the antibody have high specificity, and after being binded to an antigen, the antibody and the antigen-binding fragment thereof can enhance an immune killing function of T cells, can induce activation of immune cells and promote secretion of cytokines, and can significantly inhibit the growth of tumors in the body.

## Description

### Field of the Invention

The invention relates to the research field of an antibody and the humanization transformation of the antibody, and in particular, the invention relates to an antibody capable of specifically binding to 4-1BB and an antigen-binding fragment thereof.

### Background of the Invention

Monoclonal antibodies are highly target-specific antibodies that only act on a single antigenic epitope, and have been widely used in the treatment of many diseases, such as cancers, inflammatory and autoimmune diseases, infectious diseases, etc., especially when the chemotherapy of a cancer fails, this target-specific drug is especially important.

4-1BB is a co-stimulatory molecule belonging to TNFRSF. It was discovered in the late 1980s when screening T cell factors, and stimulating mouse helper T cells and cytotoxic T cells with concanavalin A. Human 4-1BBL was first isolated from the activated CD4+ T lymphocyte population by direct expression cloning in 1994. 4-1BBL is mainly expressed on dendritic cells, B cells or macrophages. Studies have shown that the ligand-receptor (such as CD40-CD40L, CD27-CD70 or OX40-OX40L) signaling pathways of TNFSF-TNFRSF can regulate many important processes in the body, such as cell development and death, cytokines and chemokines induction. More and more evidences indicate that members of TNFRSF and TNFSF are involved in inflammations and pathologies of various diseases including cancers. In immunotherapies, an effective immune response requires two biological signals to adequately activate T cells and other immune cells. The first signal, i.e. an antigen-specific signal, is produced by the interaction of lymphocyte receptors with specific peptides bound to major histocompatibility complex (MHC) molecules on antigen-presenting cells (APCs). The second signal is a non-antigen-specific co-stimulatory signal. This type of signal is provided by the interaction between T cells and co-stimulatory molecules expressed on APCs.

Therefore, immunotherapeutic technologies targeting TNFSF and TNFRSF ligand-receptor interactions have high potential in cancer treatments. There remains a need in the art to develop more potent antibodies specifically binding to 4-1BB.

### Summary of the Invention

A first aspect of the invention relates to an isolated anti-human 4-1BB antibody, an antigen-binding fragment, a variant, or a derivative thereof, wherein the antibody or antigen-binding fragment thereof comprises three complementarity determining region LCDRs of the light chain variable region set forth in SEQ ID No. 7, and three complementarity determining region HCDRs of the heavy chain variable region set forth in SEQ ID No. 8.

The antibody or antigen-binding fragment thereof comprises a light chain variable region and/or a heavy chain variable region, wherein
the light chain variable region comprises a LCDR1 of the amino acid sequences set forth in SEQ ID No. 1, a LCDR2 of the amino acid sequences set forth in SEQ ID No. 2, and a LCDR3 of the amino acid sequences set forth in SEQ ID No. 3; and/or
the heavy chain variable region comprises a HCDR1 of the amino acid sequences set forth in SEQ ID No. 4, a HCDR2 of the amino acid sequences set forth in SEQ ID No. 5, and a HCDR3 of the amino acid sequences set forth in SEQ ID No. 6.

The amino acid sequences of the CDRs of the light chain variable region or the heavy chain variable region have at least 70% identity to the sequences set forth in SEQ ID Nos. 1-3 and 4-6 respectively and retain the biological activity of the corresponding parent sequences, such as at least 75%, 80%, 85%, 90% or 95% or higher identity; or
the amino acid sequences of the CDRs of the light chain variable region or the heavy chain variable region are variant sequences of the sequences set forth in SEQ ID Nos: 1-3 and 4-6 respectively obtained after deletion, substitution and/or addition of one or more amino acid residues which retain the biological activities of the corresponding parent sequences, such as 1, 2, 3 or more amino acid residues.

In some embodiments, the variant is selected from the group consisting of chimeric antibodies, humanized antibodies, and fully human antibodies.

In some embodiments, the heavy chain constant region sequence of the antibody is the constant region sequence of one selected from the group consisting of human IgG1, IgG2, IgG3, IgG4, IgA, IgM, IgE, IgD, and/or, the light chain constant region sequence of the antibody is the constant region sequence of κ chain or λ chain; preferably, the heavy chain constant region sequence is the constant region sequence of IgG1 or IgG4, and/or the light chain constant region sequence is the constant region sequenceof κ light chain.

In some embodiments, the amino acid sequence of the light chain variable region of a chimeric anti-4-1BB antibody or a functional fragment thereof is as set forth in SEQ ID NO. 7; or has at least 70% identity to the sequence set forth in SEQ ID No. 7 and retains the biological activity of the corresponding parent sequence, such as at least 75%, 80%, 85%, 90%, 95%, 96%, 97%, 98%, 99% % or higher identity; or is a variant sequence of the sequence set forth in SEQ ID NO. 7 obtained by deleting, substituting and/or adding one or more amino acid residues which retains the biological activity of the corresponding parent sequence, such as 1, 2, 3, 4, 5, 6, 7, 8, 9, 10, 15, 20 or more amino acid residues; and/or the amino acid sequence of the heavy chain variable region is as set forth in SEQ ID NO. 8; or has at least 70% identity to the sequence set forth in SEQ ID NO. 8 and retains the biological activity of corresponding parent sequence, such as at least 75%, 80%, 85%, 90%, 95%, 96%, 97%, 98%, 99% or higher identity; or is a variant sequence of the sequence set forth in SEQ ID NO. 8 obtained by deleting, substituting and/or adding one or more amino acid residues that retains the biological activity of the corresponding parent sequence, such as 1, 2, 3, 4, 5, 6, 7, 8, 9, 10, 15, 20 or more amino acid residues.

In some embodiments, the chimeric anti-4-1BB antibody or a functional fragment thereof comprises a light chain variable region and a heavy chain variable region, wherein:
(a) the light chain variable region has the amino acid sequence selected from those set forth in SEQ ID Nos. 13-20; and/or
   the heavy chain variable region has the amino acid sequence selected from those set forth in SEQ ID Nos. 21-31;
(b) the light chain variable region has the amino acid sequence set forth in SEQ ID No. 11; and/or
   the heavy chain variable region has the amino acid sequence selected from those set forth in SEQ ID Nos. 21-31;
(c) the light chain variable region has the amino acid sequence selected from those set forth in SEQ ID Nos. 13-20; and/or
   the heavy chain variable region has the amino acid sequence set forth in SEQ ID No. 12.

Preferably, the light chain variable region has the amino acid sequence set forth in SEQ ID No. 11; and/or the heavy chain variable region has the amino acid sequence set forth in SEQ ID No. 12.

In some embodiments, the antigen-binding fragment is one or more selected from the group consisting of F(ab')₂, Fab', Fab, Fd, Fv, scFv, diabody, camelid antibody, CDR, and antibody minimal recognition unit (dAb), preferably, the antigen-binding fragment is Fab, F(ab')₂ or scFv.

A second aspect of the invention relates to an isolated nucleic acid molecule selected from the group consisting of:
(1) DNA or RNA encoding the anti-human 4-1BB antibody, antigen-binding fragment, variant, or derivative thereof as described in the first aspect;
(2) a nucleic acid that is completely complementary to the DNA or RNA defined in (1).

A third aspect of the invention relates to a vector comprising the nucleic acid molecule as described in the second aspect operably linked, preferably, the vector is an expression vector.

A fourth aspect of the invention relates to a host cell comprising the nucleic acid molecule as described in the second aspect or the vector as described in the third aspect.

A fifth aspect of the invention relates to a composition comprising the anti-human 4-1BB antibody, antigen-binding fragment, variant or derivative thereof as described in the first aspect, the nucleic acid molecule as described in the second aspect, the vector as described in the third aspect or the host cell as described in the fourth aspect, and a pharmaceutically acceptable excipient.

A sixth aspect of the invention relates to a method of producing the anti-human 4-1BB antibody, antigen-binding fragment, variant, or derivative thereof as described in the first aspect, comprising the steps of:
culturing the host cells as described in the fourth aspect under culture conditions suitable for the expression of the anti-human 4-1BB antibody, antigen-binding fragment or variant thereof, and optionally, isolating and purifying the obtained products, and optionally, conjugating the resultant products with a diagnostic and/or therapeutic agent.

A seventh aspect of the invention relates to use of the anti-human 4-1BB antibody, antigen-binding fragment, variant or derivative thereof as described in the first aspect, the nucleic acid molecule as described in the second aspect, the vector as described in the third aspect or the host cell as described in the fourth aspect in the manufacture of a medicament for the prevention and/or the treatment of autoimmune diseases, immune responses against grafts, allergies, infectious diseases, neurodegenerative diseases and tumors.

An eighth aspect of the invention relates to the anti-human 4-1BB antibody, antigen-binding fragment, variant or derivative thereof as described in the first aspect, the nucleic acid molecule as described in the second aspect, the vector as described in the third aspect or the host cell as described in the fourth aspect, for use in the prevention and/or treatment of 4-1BB-mediated diseases or disorders, wherein the diseases or disorders are preferably tumors; more preferably, the tumors are one or more selected from the group consisting of leukemia, lymphoma, myeloma, brain tumor, head and neck squamous cell carcinoma, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell carcinoma, and melanoma.

A ninth aspect of the invention relates to a method of preventing and/or treating tumors, comprising administering to a subject in need thereof the anti-human 4-1BB antibody, antigen-binding fragment, variant or derivative thereof as described in the first aspect, the nucleic acid molecule as described in the second aspect, the vector as described in the third aspect or the host cell as described in the fourth aspect.

In some embodiments, the tumors are one or more selected from the group consisting of leukemia, lymphoma, myeloma, brain tumor, head and neck squamous cell carcinoma, non-small cell lung cancer, small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, urothelial cancer, renal cell carcinoma, osteosarcoma, melanoma, and merkel cell carcinoma; preferably, the tumors are one or more selected from the group consisting of lymphoma, myeloma, head and neck squamous cell carcinoma, non-small cell lung cancer, small cell lung cancer, nasopharyngeal cancer, esophageal cancer, gastric cancer, liver cancer, colorectal cancer, breast cancer, cervical cancer, endometrial cancer, prostate cancer, urothelial cancer, renal cell carcinoma, osteosarcoma, melanoma, and merkel cell carcinoma; more preferably, the tumors are one or more selected from the group consisting of lymphoma, head and neck squamous cell carcinoma, non-small cell lung cancer, gastric cancer, liver cancer, colorectal cancer, cervical cancer, urothelial carcinoma, renal cell carcinoma, and melanoma, and merkel cell carcinoma.

In some embodiments, the subject is selected from mammals, including but not limited to human and/or other primates; mammals, including commercially relevant mammals such as cattle, pigs, horses, sheep, cats, dogs, mice and/or rats.

In some embodiments, the anti-human 4-1BB antibody, antigen-binding fragment, variant or derivative thereof, the nucleic acid molecule, the vector or the host cell of the invention is utilized by conventional administration methods in the art, such as parenteral route, such as intravenous injection.

The anti-4-1BB monoclonal antibody of the invention has strong specificity, good stability, strong activity of regulating T cell function and good pharmacokinetic properties, and can significantly inhibit tumor growth *in vivo.* Moreover, the activity of the anti-4-1BB monoclonal antibody of the invention of blocking 4-1BB/4-1BBL is stronger than that of Urelumab.

### Brief description of the figures

To illustrate the embodiments of the invention or the technical solutions in the prior art more clearly, the following will briefly introduce the accompanying drawings that need to be used in the embodiments or the prior art. Apparently, the accompanying drawings in the following description show some embodiments of the invention, and for those skilled in the art, they can obtain other accompanying drawings based on these accompanying drawings without any creative work.
Figure 1. Panel A shows the binding activity of the chimeric anti-human 4-1BB monoclonal antibody sequence to 4-1BB, and panel B shows the agonistic activity of the chimeric anti-human 4-1BB monoclonal antibody sequence to 4-1BB.
Figure 2 shows the binding activity of the chimeric anti-human 4-1BB monoclonal antibody mAb-33-83 to 4-1BB.
Figure 3. Panel A shows the species specificity of the chimeric anti-human 4-1BB monoclonal antibody mAb-33-83, and panel B shows the binding specificity of the chimeric anti-human 4-1BB monoclonal antibody mAb-33-83.
Figure 4 shows the activity of the chimeric anti-human 4-1BB monoclonal antibody mAb-33-83 of blocking the binding to 4-1BB/4-1BBL.
Figure 5 shows the anti-tumor efficacy of the chimeric anti-human 4-1BB monoclonal antibody mAb-33-83 in a gene knock-in mouse homologous tumor model.
Figure 6 shows the binding activity of the anti-human 4-1BB humanized monoclonal antibody BH3145b to 4-1BB.
Figure 7 shows the activity of the anti-human 4-1BB humanized monoclonal antibody BH3145b of blocking the binding to 4-1BB/4-1BBL.

### Detailed description of the embodiments

### Definitions

The term "4-1BB", i.e. CD137, is a member of the tumor necrosis factor receptor superfamily (TNFRSF9), mainly expressed on activated T cells, and is a co-stimulatory molecule of T cells, and its ligand is 4-1BBL.

As used herein, the terms "anti-4-1BB antibody", "anti-4-1BB", "4-1BB antibody", "anti-4-1B mAb" or "antibody binding to 4-1BB" refer to an antibody which is capable of binding to the 4-1BB protein or a fragment thereof with sufficient affinity. In some embodiments, the anti-4-1BB antibody binds to an epitope of 4-1BB that is conserved among 4-1BBs from different species.

The term "antibody" refers to an immunoglobulin molecule or a fragment of an immunoglobulin molecule which can bind to an epitope of an antigen. Naturally occurring antibodies typically comprise a tetramer which is usually composed of at least two heavy (H) chains and at least two light (L) chains. Immunoglobulins include the following isotypes: IgG (IgG1, IgG2, IgG3, and IgG4 subtypes), IgA (IgA₁ and IgA₂ subtypes), IgM, and IgE, with their corresponding heavy chains being the µ, δ, γ, α, and ε chains, respectively. Light chains can be divided into κ and λ chains according to the constant regions.

Herein, the term "antibody" is used in the broadest sense thereof, and refers to a protein comprising an antigen-binding site, encompassing natural and artificial antibodies of various structures, including but not limited to intact antibodies and antigen-binding fragments of antibodies.

A "variable region" or "variable domain" is a domain of a heavy or light chain of an antibody involving in the binding of the antibody to its antigen. Each heavy chain of an antibody consists of a heavy chain variable region (herein referred to as VH) and a heavy chain constant region (herein referred to as CH), wherein the heavy chain constant region usually consists of 3 domains (CH1, CH2 and CH3). Each light chain consists of a light chain variable region (herein referrdd to as VL) and a light chain constant region (herein referrdd to as CL). The variable regions of the heavy and light chains are typically responsible for antigen recognition, while the constant domains of the heavy and light chains can mediate the binding of the immunoglobulin with host tissues or factors (including various cells of the immune system (e.g., effector cells), Fc receptors and the first component of the classical complement system (C1q)). The heavy and light chain variable regions comprise binding regions interacting with an antigen. The VH and VL regions can be further subdivided into hypervariable regions (HVRs) called "complementarity determining regions (CDRs)", which are interspersed with more conserved regions called "framework regions (FRs)". Each VH or VL consists of three CDR domains and four FR domains, arranged from the amino-terminus to the carboxy-terminus in the following order: FR1 - CDR1 - FR2 - CDR2 - FR3 - CDR3 - FR4.

The terms "complementarity determining region" or "CDR region" or "CDR" (used interchangeably herein with the hypervariable region "HVR") refer to a region in the antibody variable domains which is hypervariable in sequence and forms a loop structurally determined ("a hypervariable loop") and/or comprises antigen-contacting residues ("antigen-contacting points"). The CDRs are primarily responsible for binding to an epitope. Herein, the three CDRs of the heavy chain are referred to as HCDR1, HCDR2 and HCDR3, and the three CDRs of the light chain are referred to as LCDR1, LCDR2 and LCDR3.

It should be noted that the boundaries of the CDRs of the variable regions of the same antibody obtained based on different numbering systems may be different from each other. That is, the CDR sequences of the variable regions of the same antibody defined under different numbering systems are somewhat different from each other. Thus, when defining an antibody with the particular CDR sequences as defined in the invention, the scope of said antibody also covers antibodies of which the variable region sequences comprise said particular CDR sequences, but due to the different numbering system(s) used (such as different numbering system rules or combinations thereof), the CDR boundaries claimed by them might be different from the specific CDR boundaries defined in the invention.

The terms "mAbs", "monoclonal antibodies "or "monoclonal antibody composition" refer to a preparation of antibody molecules of single molecular composition, and refer to antibodies obtained from a substantially homogeneous population of antibodies, i.e., the population comprising individual antibodies are identical except for the naturally occurring mutations that may be present in minor amounts. A conventional monoclonal antibody compositions displays a single binding specificity and affinity for a particular epitope. In certain embodiments, a monoclonal antibody can be composed of more than one Fab domain thereby increasing the specificity to more than one target. The term "monoclonal antibody" or "monoclonal antibody composition" is not limited by any particular method of production (eg, recombinant, transgenic, hybridoma, etc.).

The terms "double antibody", "bifunctional antibody", "bispecific antibody" or "BsAb" refers to an antibody which has two different antigen binding sites, so that which can simultaneously bind to two target antigens, alternatively which plays a role in mediating another special function simultaneously besides the targeting effect of the antibody. The special function effector molecules mediated can also be toxins, enzymes, cytokines, radionuclides, etc. The two arms of the bispecific antibody binding to an antigen can be from Fab, Fv, ScFv or dSFv, etc.

The term "polyclonal antibody" refers to a preparation comprising different antibodies raised against different determinants ("epitopes").

The term "antigen-binding fragment of antibody" refers to a fragment, a part, a region or a domain of an antibody capable of binding to an epitope (e.g., obtainable by cleavage, recombination, synthesis, etc.). An antigen-binding fragment may comprise 1, 2, 3, 4, 5 or all 6 CDR domains of such antibodies and, while capable of binding to the epitope, it may exhibit different specificities, affinities or selectivities. Preferably, an antigen-binding fragment comprises all 6 CDR domains of said antibody. An antigen-binding fragment of an antibody may be a part of a single polypeptide chain (e.g., scFv) or comprise a single polypeptide chain, or may be a part of two or more polypeptide chains (each having an amino- and carboxy-terminus) (e.g., a bispecific antibody, a Fab fragment, a F (ab')₂ fragment, etc.) or comprise two or more polypeptide chains.

Examples of an antigen-binding fragment of the invention include (a) a Fab' or Fab fragment, a monovalent fragment consisting of the VL, VH, CL and CH1 domains; (b) F(ab')₂ fragments, bivalent fragments comprising two Fab fragments linked by a disulfide bond at the hinge domain; (c) an Fd fragment consisting of the VH and CH1 domains; (d) a Fv fragment consisting of VL and VH domains of a single arm of an antibody; (e) a single chain Fv (scFv), a recombinant protein formed by linking VH and VL domains of an antibody with a peptide linker by genetic engineering; (f) a dAb fragment (Ward et al., Nature, 341, 544 -546 (1989)), which consists essentially of a VH domain and also called domain antibodies (Holt et al., Trends Biotechnol., 2i(11): 484-90); (g) camelid or nanobodies (Revets et al., Expert Opin Biol Ther., 5(1): 111-24) and (h) an isolated complementarity determining region (CDR).

The term "chimeric antibody" means that a portion of the heavy and/or light chains is identical or homologous to the corresponding sequences of an antibody from a particular species or belonging to a particular antibody class or subclass, while the rest of the chains is identical or homologous to the corresponding sequences of an antibody from another species or belonging to another antibody class or subclass, providing that they exhibit the desired biological activity. The invention provides the variable region antigen binding sequences from human antibodies. Thus, chimeric antibodies primarily focused herein include antibodies which comprise one or more human antigen-binding sequences (e.g., CDRs) and comprise one or more sequences from non-human antibodies, such as FR or C region sequences. Furthermore, chimeric antibodies described herein are antibodies comprising human variable region antigen-binding sequence from one antibody class or subclass and other sequences from another antibody class or subclass, such as FR or C region sequences.

The term "humanized antibody" refers to an antibody in which CDR sequences from the germline of another mammalian species, such as a mouse, have been grafted among the human framework region sequences. Additional framework region modifications can be made within the human framework region sequences.

The term "human antibody" or "fully human antibody" ("humAb "or "HuMab ") refers to an antibody comprising variable and constant domains derived from human germline immunoglobulin sequences. The human antibodies of the invention may include amino acid residues not encoded by human germline immunoglobulin sequences (e.g., mutations introduced by random or site-specific mutagenesis *in vitro* or during gene rearrangement or by somatic mutation *in vivo*).

Variant antibodies are also included within the scope of the invention. Accordingly, variants of the sequences recited in the invention are also included within the scope of the invention. Other variants of antibody sequences with improved affinity can be obtained by using methods known in the art and these variants are also included within the scope of the invention. For example, amino acid substitutions can be used to obtain antibodies with further improved affinity. Alternatively, codon optimization of nucleotide sequences can be used to improve the translation efficiency of expression systems in antibody production.

Such variant antibody sequences have 70% or more (e.g., 80%, 85%, 90%, 95%, 97%, 98%, 99% or more) sequence homology to the sequences recited in the invention. Such a sequence homology is obtained by calculation relative to the full length of the reference sequence (i.e., the sequence recited in the invention).

The numbering of amino acid residues in the invention is according to IMGT^{®}, the international ImMunoGeneTics information system^{®} or Kabat, E. A., Wu, T. T., Perry, H. M., Gottesmann, K. S. & Foeller, C. (1991). Sequences of Proteins of Immunological Interest, 5th edit., NIH Publication no. 91-3242 U.S. Department of Health and Human Services; Chothia, C. & Lesk, A. M. (1987). Canonical structures For The Hypervariable domains Of Immunoglobulins. J. Mol. Biol. 196, 901-917. Unless otherwise specified, the numbering of amino acid residues in the invention is according to the Kabat numbering system.

An antibody or an antigen-binding fragment thereof is said to "specifically" bind a region of another molecule (*i.e.,* an epitope) if it reacts or associates more frequently, more rapidly, with greater duration and/or with greater affinity or avidity with that epitope relative to alternative epitopes. In some embodiments, the antibody or antigen-binding fragment thereof of the invention binds human 4-1BB4-1BB with an affinity of at least 10⁻⁷ M, such as 10⁻⁸ M, 10⁻⁹ M, 10⁻¹⁰ M, 10⁻¹¹ M or higher. Preferably, the antibody or antigen-binding fragment thereof binds under physiological conditions (e.g., *in vivo*). Therefore, "specifically binding to 4-1BB" refers to the ability of the antibody or antigen-binding fragment thereof to bind to 4-1BB with the above-mentioned specificity and/or under such conditions. Methods suitable for determining such a binding are known in the art.

The term "binding" in the context of the binding of an antibody to a predetermined antigen typically refers to binding with an affinity corresponding to a KD of about 10⁻⁶ M or less, which is at least ten-fold lower, such as at least 100-fold lower, for instance at least 1,000-fold lower than its affinity for binding to a non-specific antigen (e.g., BSA, casein) other than the predetermined antigen or a closely-related antigen.

As used herein, the term "kd" (sec -1 or 1/s) refers to the dissociation constant of a particular antibody-antigen interaction. Said value is also referred as the k_{off} value.

As used herein, the term "ka" (M-1 x sec-1 or 1/Msec) refers to the association rate constant of a particular antibody-antigen interaction.

As used herein, the term "KD" (M) refers to the dissociation equilibrium constant of a particular antibody-antigen interaction and is obtained by dividing the kd by the ka.

As used herein, the term "KA" (M-1 or 1/M) refers to the association equilibrium constant of a particular antibody-antigen interaction and is obtained by dividing the ka by the kd.

In some embodiments, only part of a CDR, namely the subset of CDR residues required for binding, termed the SDRs, are needed to retain binding in a humanized antibody. CDR residues not contacting the relevant epitope and not in the SDRs can be identified based on previous studies (for example residues H60-H65 in CDR H2 are often not required), from regions of Kabat CDRs lying outside Chothia hypervariable loops (see, Kabat et al. (1992), "Sequences of Proteins of Immunological Interest", National Institutes of Health Publication No. 91-3242; Chothia, C. et al. (1987), "Canonical Structures For The Hypervariable Regions Of Immunoglobulins", J. Mol. Biol. 196:901-917), by molecular modeling and/or empirically, or as described in Gonzales, N.R. et al. (2004), "SDR Grafting Of A Murine Antibody Using Multiple Human Germline Templates To Minimize Its Immunogenicity", Mol. Immunol. 41:863-872. In such humanized antibodies at positions in which one or more donor CDR residues is absent or in which an entire donor CDR is omitted, the amino acid occupying the position can be an amino acid occupying the corresponding position (by Kabat numbering) in the acceptor antibody sequence. The number of such substitutions of acceptor for donor amino acids in the CDRs to include reflects a balance of competing considerations. Such substitutions are potentially advantageous in decreasing the number of mouse amino acids in a humanized antibody and consequently decreasing potential immunogenicity. However, substitutions can also cause changes of affinity, and significant reductions in affinity are preferably avoided. Positions for substitution within CDRs and amino acids to substitute can also be selected empirically.

The fact that a single amino acid alteration of a CDR residue can result in loss of functional binding (Rudikoff, S. et al. (1982), "Single Amino Acid Substitution Altering Antigen-Binding Specificity", Proc. Natl. Acad. Sci. (USA) 79(6): 1979-1983) provides a means for systematically identifying alternative functional CDR sequences. In one preferred method for obtaining such variant CDRs, a polynucleotide encoding the CDR is mutagenized (for example via random mutagenesis or by a site-directed method (e.g., polymerase chain-mediated amplification with primers that encode the mutated locus)) to produce a CDR having a substituted amino acid residue. By comparing the identity of the relevant residue in the original (functional) CDR sequence to the identity of the substituted (non-functional) variant CDR sequence, the BLOSUM62.iij substitution score for that substitution can be identified. The BLOSUM system provides a matrix of amino acid substitutions created by analyzing a database of sequences for trusted alignments (Eddy, S.R. (2004), "Where Did The BLOSUM62 Alignment Score Matrix Come From?", Nature Biotech. 22(8): 1035-1036; Henikoff, J.G. (1992), "Amino acid substitution matrices from protein blocks", Proc. Natl. Acad. Sci. (USA) 89: 10915-10919; Karlin, S. et al. (1990), "Methods For Assessing The Statistical Significance Of Molecular Sequence Features By Using General Scoring Schemes", Proc. Natl. Acad. Sci. (USA) 87:2264-2268; Altschul, S.F. (1991), "Amino Acid Substitution Matrices From An Information Theoretic Perspective", J. Mol. Biol. 219, 555-565. Currently, the most advanced BLOSUM database is the BLOSUM62 database (BLOSUM62.iij). **Table 1** presents the BLOSUM62.iij substitution scores (the higher the score the more conservative the substitution and thus the more likely the substitution will not affect function). If an epitope-binding fragment comprising the resultant CDR fails to bind to 4-1BB, for example, then the BLOSUM62.iij substitution score is deemed to be insufficiently conservative, and a new candidate substitution is selected and produced having a higher substitution score. Thus, for example, if the original residue was glutamate (E), and the non-functional substitute residue was histidine (H), then the BLOSUM62.iij substitution score will be 0, and more conservative changes (such as to aspartate, asparagine, glutamine, or lysine) are preferred.

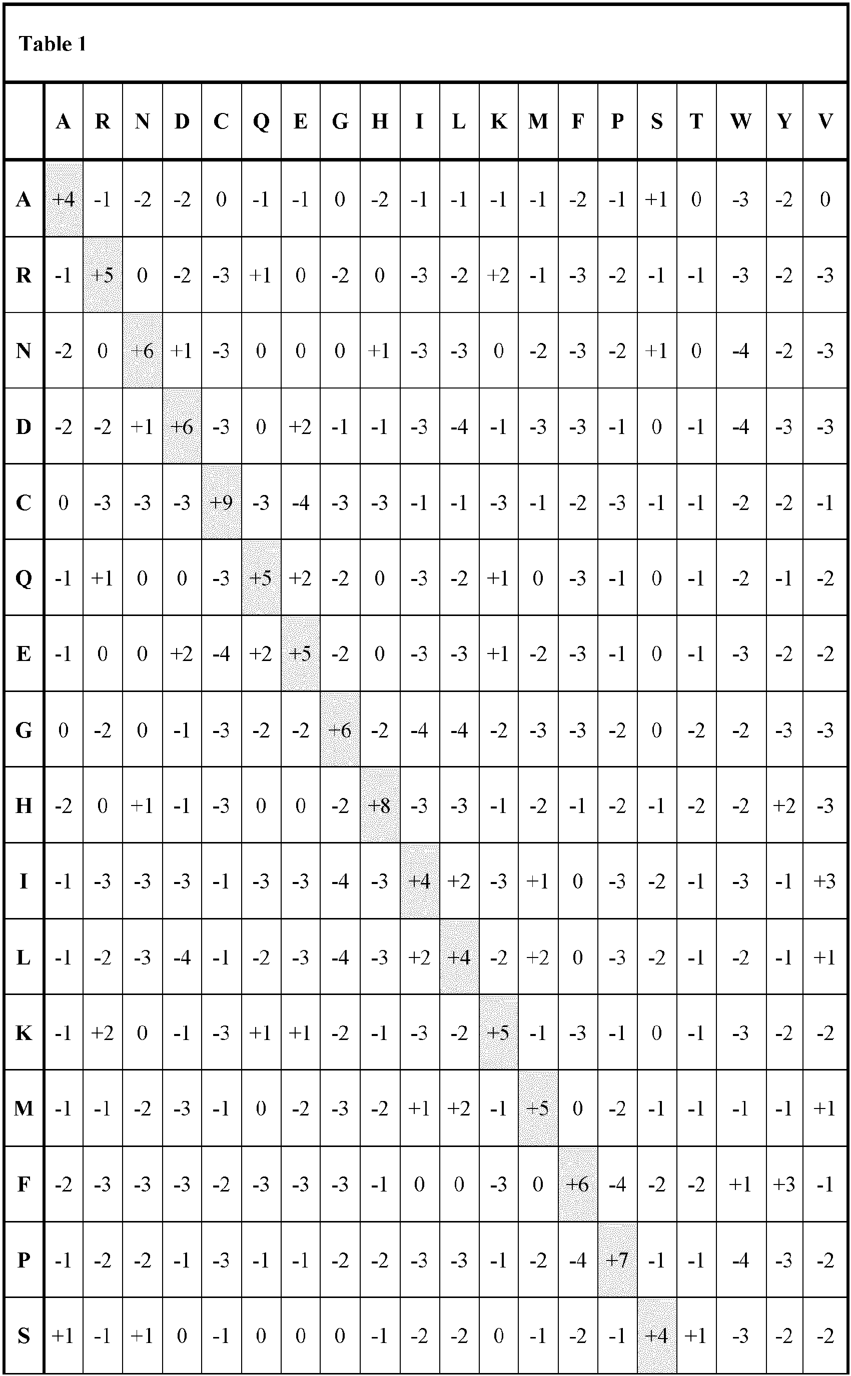

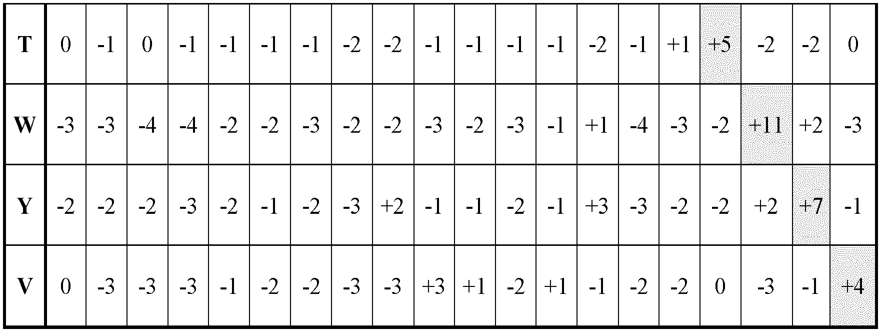

The invention thus contemplates the use of random mutagenesis to identify improved CDRs. In the context of the present invention, conservative substitutions may be defined by substitutions within the classes of amino acids reflected in one or more of **Tables 2, 3, or 4:**

### Amino Acid Residue Classes For Conservative Substitutions:

| **Table 2** | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Cly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

### Alternative Conservative Amino Acid Residue Substitution Classes:

| **Table 3** | | | |
|---|---|---|---|
| **1** | **A** | **S** | **T** |
| **2** | **D** | **E** | |
| **3** | **N** | **Q** | |
| **4** | **R** | **K** | |
| **5** | **I** | **L** | **M** |
| **6** | **F** | **Y** | **W** |

### Alternative Physical and Functional Classifications of Amino Acid Residues:

| | **Table 4** | |
|---|---|---|
| Alcohol Group-Containing Residues | | S and T |
| Aliphatic Residues | | I, L, V and M |
| Cycloalkenyl-Associated Residues | | F, H, W and Y |
| Hydrophobic Residues | | A, C, F, G, H, I, L, M, R, T, V, W and Y |
| Negatively Charged Residues | | D and E |
| Polar Residues | | C, D, E, H, K, N, Q, R, S and T |
| Positively Charged Residues | | H, K and R |
| Small Residues | | A, C, D, G, N, P, S, T and V |
| Very Small Residues | | A, G and S |
| Residues Involved In Turn Formation | | A, C, D, E, G, H, K, N, Q, R, S, P and T |
| Flexible Residues | | Q, T, K, S, G, P, D, E and R |

More conservative substitutions groupings include: valine-leucine-isoleucine, phenylalanine-tyrosine, lysine-arginine, alanine-valine, and asparagine-glutamine.

In some embodiments, the hydrophilic amino acid is selected from the group consisting of Arg, Asn, Asp, Gln, Glu, His, Tyr, and Lys.

Additional groups of amino acids may also be formulated using the principles described in, e.g., Creighton (1984) Proteins: Structure and Molecular Properties (2d Ed. 1993), W. H. Freeman and Company.

Thus, the sequences of the CDR variants comprised in antibodies or antigen-binding fragments thereof may differ from those of the CDRs of the parent antibody by substitutions, such as substitutions of 4, 3, 2 or 1 amino acid residues. According to an embodiment of the invention, amino acid(s) in the CDR regions may be substituted with conservative substitutions, as defined in 3 Tables above.

"Homology" or "sequence identity" refers to the percentage of residues in a variant polynucleotide or polypeptide sequence that are identical to those of the non-variant sequence, after alignment of the sequences and introduction of gaps. In specific embodiments, polynucleotide and polypeptide variants have at least about 70%, at least about 75%, at least about 80%, at least about 90%, at least about 95%, at least about 98%, or at least about 99% polynucleotide or polypeptide homology.

Such variant polypeptide sequences have 70% or higher (i.e., 80%, 85%, 90%, 95%, 97%, 98%, 99% or higher) sequence identity to the sequences recited in the invention. In other embodiments, the invention provides polypeptide fragments comprising contiguous stretches of various lengths of the amino acid sequences disclosed herein. For example, where applicable, the peptide sequences provided herein comprise at least about 5, 10, 15, 20, 30, 40, 50, 75, 100, 150 or more, and all intermediate lengths therebetween, contiguous amino acid residues of one or more sequences disclosed herein.

The term "treatment" or "treating" as used herein means ameliorating, slowing, attenuating, or reversing the progress or severity of a disease or disorder, or ameliorating, slowing, attenuating, or reversing one or more symptoms or side effects of such disease or disorder. In this invention, "treatment" or "treating" further means an approach for obtaining beneficial or desired clinical results, where "beneficial or desired clinical results" include, without limitation, alleviation of a symptom, diminishment of the extent of a disorder or disease, stabilized (i.e., not worsening) disease or disorder state, delay or slowing of the progression a disease or disorder state, amelioration or palliation of a disease or disorder state, and remission of a disease or disorder, whether partial or total, detectable or undetectable.

The term "prevention" or "preventing" refers to the administration of antibodies and functional fragments thereof of the invention, thereby preventing or retarding the development of at least one symptom of a disease or condition. The term also includes treating a subject in remission to prevent or hinder relapse.

Antibodies of the invention may be monoclonal antibodies produced by recombinant DNA techiniques.

The antibody of the invention may be of any isotype. The choice of isotype typically will be guided by the desired effector functions, such as ADCC induction. Exemplary isotypes are IgG1, IgG2, IgG3, and IgG4. Either of the human light chain constant domains, κ or λ, may be used. If desired, the class of an anti-4-1BB antibody of the present invention may be switched by known methods. For example, an antibody of the present invention that was originally IgG may be class switched to an IgM antibody of the present invention. Further, class switching techniques may be used to convert one IgG subclass to another, for instance from IgG1 to IgG2. Thus, the effector function of the antibodies of the present invention may be changed by isotype switching to, e.g., an IgG1, IgG2, IgG3, IgG4, IgD, IgA, IgE, or IgM antibody for various therapeutic uses. In one embodiment an antibody of the present invention is an IgG4 antibody. An antibody is said to be of a particular isotype if its amino acid sequence is most homologous to that isotype, relative to other isotypes.

In some embodiments, the antibodies of the invention are full-length antibodies, preferably IgG antibodies. In other embodiments, the antibodies of the invention are antibody antigen-binding fragments or single chain antibodies.

In some embodiments, the anti-4-1BB antibody is a monovalent antibody, preferably a monovalent antibody as described in WO2007059782 (which is incorporated herein by reference in its entirety) having a deletion of the hinge region. Accordingly, in some embodiments, the antibody is a monovalent antibody, wherein said anti-4-1BB antibody is constructed by a method comprising: i) providing a nucleic acid construct encoding the light chain of said monovalent antibody, said construct comprising a nucleotide sequence encoding the VL region of a selected antigen specific anti-4-1BB antibody and a nucleotide sequence encoding the constant CL region of an Ig, wherein said nucleotide sequence encoding the VL region of a selected antigen specific antibody and said nucleotide sequence encoding the CL region of an Ig are operably linked together, and wherein, in case of an IgG1 subtype, the nucleotide sequence encoding the CL region has been modified such that the CL region does not contain any amino acids capable of forming disulfide bonds or covalent bonds with other peptides comprising an identical amino acid sequence of the CL region in the presence of polyclonal human IgG or when administered to an animal or human being; ii) providing a nucleic acid construct encoding the heavy chain of said monovalent antibody, said construct comprising a nucleotide sequence encoding the VH region of a selected antigen specific antibody and a nucleotide sequence encoding a constant CH region of a human Ig, wherein the nucleotide sequence encoding the CH region has been modified such that the region corresponding to the hinge region and, as required by the Ig subtype, other regions of the CH region, such as the CH3 region, does not comprise any amino acid residues which participate in the formation of disulphide bonds or covalent or stable non-covalent inter-heavy chain bonds with other peptides comprising an identical amino acid sequence of the CH region of the human Ig in the presence of polyclonal human IgG or when administered to an animal human being, wherein said nucleotide sequence encoding the VH region of a selected antigen specific antibody and said nucleotide sequence encoding the CH region of said Ig are operably linked together; iii) providing a cell expression system for producing said monovalent antibody; iv) producing said monovalent antibody by co-expressing the nucleic acid constructs of (i) and (ii) in cells of the cell expression system of (iii).

Similarly, in some embodiments, the anti-4-1BB antibody of the invention is a monovalent antibody, which comprises:
(i) a variable domain of an antibody of the invention as described herein or an epitope-binding part of the said domain, and
(ii) a CH domain of an immunoglobulin or a domain thereof comprising the CH2 and CH3 domains, wherein the CH domain or domain thereof has been modified such that the domain corresponding to the hinge domain and, if the immunoglobulin is not an IgG4 subtype, other domains of the CH domain, such as the CH3 domain, do not comprise any amino acid residues, which are capable of forming disulfide bonds with an identical CH domain or other covalent or stable non-covalent inter-heavy chain bonds with an identical CH domain in the presence of polyclonal human IgG.

In some other embodiments, the heavy chain of the monovalent antibody of the invention has been modified such that the entire hinge region has been deleted.

In other embodiments, the sequence of the monovalent antibody has been modified so that it does not comprise any acceptor sites for N-linked glycosylation.

The invention also includes "Bispecific Antibodies," wherein an anti-4-1BB binding region (e.g., a 4-1BB-binding region of an anti-4-1BB monoclonal antibody) is part of a bivalent or polyvalent bispecific scaffold that targets more than one epitope, (for example a second epitope could comprise an epitope of an active transport receptor, such that the bispecific antibody would exhibit improved transcytosis across a biological barrier, such as the Blood Brain Barrier, or the second epitope is an epitope of another target protein). Thus, in other embodiments, the monovalent Fab of an anti-4-1BB antibody may be joined to an additional Fab or scfv that targets a different protein to generate a bispecific antibody. A bispecific antibody can have a dual function, for example a therapeutic function imparted by an anti-4-1BB binding domain and a transport function that can bind to a receptor molecule to enhance transfer cross a biological barrier, such as the blood brain barrier.

Antibodies and epitope-binding fragments thereof of the invention also include single chain antibodies. Single chain antibodies are peptides in which the heavy and light chain Fv domains are connected. In some embodiment, the present invention provides a single-chain Fv (scFv) wherein the heavy and light chains in the Fv of an anti-4-1BB antibody of the present invention are joined with a flexible peptide linker (typically of about 10, 12, 15 or more amino acid residues) in a single peptide chain. Methods of producing such antibodies are described in for instance US 4,946,778, Pluckthun in The Pharmacology of Monoclonal Antibodies, vol. 113, Rosenburg and Moore eds. Springer-Verlag, New York, pp. 269-315 (1994), Bird et al., Science 242, 423-426 (1988), Huston et al., PNAS USA 85, 5879-5883 (1988) and McCafferty et al., Nature 348, 552-554 (1990). The single chain antibody may be monovalent, if only a single VH and VL are used, bivalent, if two VH and VL are used, or polyvalent, if more than two VH and VL are used.

Antibodies of the invention are produced by any technique known in the art, such as, but not limited to, any chemical, biological, genetic, or enzymatic technique, alone or in combination. In general, if knowing the amino acid sequence of a desired sequence, one skilled in the art can readily generate such an antibody by standard techniques used to generate polypeptides. For example, these antibodies can be synthesized using well-known solid-phase methods, preferably using a commercially available peptide synthesis apparatus (such as that manufactured by Applied Biosystems, Foster City, California) and following the manufacturer's instructions. Alternatively, antibodies of the invention can be synthesized by recombinant DNA techniques well known in the art. For example, after incorporating a DNA sequence encoding an antibody into an expression vector and introducing these vectors into eukaryotic or prokaryotic host cells suitable for expressing the desired antibodies, antibodies as DNA expression products can be obtained, which can then be isolated from the host cells using known techniques.

The antibodies and epitope-binding fragments thereof described herein may be modified by inclusion of any suitable number of modified amino acids and/or associations with such conjugated substituents. Suitability in this context is generally determined by the ability to retain the 4-1BB selectivity and/or the 4-1BB specificity at least substantially associated with the non-derivatized parent anti-4-1BB antibody. The inclusion of one or more modified amino acids may be advantageous in, for example, increasing polypeptide serum half-life, reducing polypeptide antigenicity, or increasing polypeptide storage stability. Amino acid(s) are modified, for example, co-translationally or post-translationally during recombinant production (e.g., N-linked glycosylation at N-X-S/T motifs during expression in mammalian cells) or modified by synthetic means. Non-limiting examples of a modified amino acid include a glycosylated amino acid, a sulfated amino acid, a prenylated (e.g., farnesylated, geranyl-geranylated) amino acid, an acetylated amino acid, an acylated amino acid, a PEGylated amino acid, a biotinylated amino acid, a carboxylated amino acid, a phosphorylated amino acid, and the like. References adequate to guide one of skill in the modification of amino acids are replete throughout the literature, see e.g., Walker (1998) Protein Protocols On CD-Rom, Humana Press, Totowa, NJ. The modified amino acid may, for instance, be selected from a glycosylated amino acid, a PEGylated amino acid, a farnesylated amino acid, an acetylated amino acid, a biotinylated amino acid, an amino acid conjugated to a lipid moiety, or an amino acid conjugated to an organic derivatizing agent.

The antibodies and antigen-binding fragments thereof of the invention may also be chemically modified by covalent conjugation to a polymer to for instance increase their circulating half-life. Exemplary polymers and methods to attach them to peptides are illustrated in for instance US 4,766,106; US 4,179,337; US 4,495,285 and US 4,609,546. Additional illustrative polymers include polyoxyethylated polyols and polyethylene glycol (PEG) (e.g., a PEG with a molecular weight of between about 1,000 and about 40,000 D, such as between about 2,000 and about 20,000 D, e.g., about 3,000-12,000 D).

The invention further relates to a fusion protein comprising the antibody (preferably the monoclonal antibody) of the invention.

The term "subject" refers to a warm-blooded animal, preferably a mammal (including humans, domestic and farm animals, zoo animals, sport animals, or pet animals such as dogs, cats, cows, horses, sheep, pigs, goats, rabbits, etc.), more preferably humans. In one embodiment, a subject may be a "patient", i.e., a warm-blooded animal, more preferably a human, who is waiting to receive or is receiving medical care or will be a subject of a medical procedure, or monitoring for the development of a disease. In one embodiment, the subject is an adult (e.g., a subject over 18 years old). In another embodiment, the subject is a child (e.g., a subject under 18 years old). In one embodiment, the subject is a male. In another embodiment, the subject is a female.

In one embodiment of the invention, a sample is a biological sample. Examples of biological samples include, but are not limited to, tissue lysates and extracts prepared from diseased tissues, body fluids, preferably blood, more preferably serum, plasma, synovial fluid, bronchoalveolar lavage fluid, sputum, lymph fluid, ascites, urine, amniotic fluid, peritoneal fluid, cerebrospinal fluid, pleural fluid, pericardial fluid, and alveolar macrophages.

In one embodiment of the invention, the term "sample" is intended to mean a sample taken from an individual prior to any analysis.

Accordingly, in some embodiments, the anti-4-1BB antibodies and antigen-binding fragments thereof of the invention include intact antibodies, such as IgG (IgG1, IgG2, IgG3 and IgG4 subtypes), IgA (IgA1 and IgA2 subtypes), IgD, IgM and IgE; antigen-binding fragments such as SDR, CDR, Fv, dAb, Fab, Fab₂, Fab', F(ab')₂, Fd, scFv, camelid antibodies or nanobodies; variant sequences of antibodies or antigen-binding fragments thereof, such as variant sequences having at least 80% sequence identity to the above antibodies or antigen-binding fragments thereof. In some embodiments, the invention also includes derivatives comprising the anti-4-1BB antibody or antigen-binding fragment thereof, such as chimeric antibodies, humanized antibodies, fully human antibodies, recombinant antibodies, bispecific antibodies, a product comprising modified amino acid(s), a product conjugated to a polymer, a product comprising a radioactive label, a product comprising a fluorescent label, a product comprising an enzymatic label, a product comprising a chemiluminescent substance, a product comprising a paramagnetic label derived from an intact antibody.

In a further aspect, the invention relates to an expression vector comprising nucleotide sequences encoding one or more polypeptide chains of the antibody or antigen-binding fragment thereof of the invention. Such expression vectors can be used to recombinantly produce the antibodies and antigen-binding fragments thereof of the invention.

An expression vector in the context of the present invention may be any suitable DNA or RNA vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In some embodiments, an anti-4-1BB antibody-encoding nucleic acid is comprised in a naked DNA or RNA vector, including, for example, a linear expression element (as described in, for instance, Sykes and Johnston, Nat Biotech 12, 355-59 (1997)), a compacted nucleic acid vector (as described in for instance US 6,077,835 and/or WO 00/70087), a plasmid vector such as pBR322, pUC 19/18, or pUC 118/119, a minimally-sized nucleic acid vector (as described in, for instance, Schakowski et al., MoI Ther 3, 793-800 (2001)), or as a precipitated nucleic acid vector construct, such as a CaPO4-precipitated construct (as described in, for instance, WO 00/46147, Benvenisty and Reshef, PNAS USA 83, 9551-55 (1986), Wigler et al., Cell 14, 725 (1978), and Coraro and Pearson, Somatic Cell Genetics 2, 603 (1981)). Such nucleic acid vectors and the usage thereof are well known in the art (see for instance US 5,589,466 and US 5,973,972). In some embodiments, the expression vector is X0GC (from the PCT publication WO2008/048037), pCDNA3.1 (ThermoFisher, catalog number V79520), pCHO1.0 (ThermoFisher, catalog number R80007).

In some embodiments, the vector is suitable for expression of anti-4-1BB antibodies or epitope-binding fragments thereof of the invention in a bacterial cell. Examples of such vectors include expression vectors such as BlueScript (Stratagene), pIN vectors (Van Heeke & Schuster, J. Biol. Chem. 264, 5503-5509 (1989), pET vectors (Novagen, Madison, Wisconsin), and the like.

An expression vector may also be a vector suitable for expression in a yeast system. Any vector suitable for expression in a yeast system may be employed. Suitable vectors include, for example, vectors comprising constitutive or inducible promoters such as alpha factor, alcohol oxidase and PGH (reviewed in: F. Ausubel et al., ed. Current Protocols in Molecular Biology, Greene Publishing and Wiley InterScience New York (1987), Grant et al., Methods in Enzymol 153, 516-544 (1987), Mattanovich, D. et al. Methods Mol. Biol. 824, 329-358 (2012), Celik, E. et al. Biotechnol. Adv. 30(5), 1108-1118 (2012), Li, P. et al. Appl. Biochem. Biotechnol. 142(2), 105-124 (2007), Böer, E. et al. Appl. Microbiol. Biotechnol. 77(3), 513-523 (2007), van der Vaart, J.M. Methods Mol. Biol. 178, 359-366 (2002), and Holliger, P. Methods Mol. Biol. 178, 349-357 (2002)).

In an expression vector of the invention, the anti-4-1BB antibody-encoding nucleic acids may comprise or be associated with any suitable promoter, enhancer, and other expression-facilitating elements. Examples of such elements include strong expression promoters (e.g., human CMV IE promoter/enhancer as well as RSV, SV40, SL3 -3, MMTV, and HIV LTR promoters), effective poly (A) termination sequences, an origin of replication for plasmid product in E. coli, an antibiotic resistance gene as selectable marker, and/or a convenient cloning site (e.g., a polylinker). Nucleic acids may also comprise an inducible promoter as opposed to a constitutive promoter such as CMV IE (the skilled artisan will recognize that such terms are actual descriptors of a degree of gene expression under certain conditions).

In an even further aspect, the invention relates to a recombinant eukaryotic or prokaryotic host cell, such as a transfectoma, which produces an antibody or epitope-binding fragment thereof of the invention as defined herein or a bispecific molecule of the invention as defined herein. Examples of host cells include yeast, bacteria, and mammalian cells, such as CHO or HEK cells. For example, in some embodiments, the present invention provides a cell comprising a nucleic acid stably integrated into the cellular genome that comprises a sequence coding for expression of an anti-4-1BB antibody of the present invention or an epitope-binding fragment thereof. In other embodiments, the present invention provides a cell comprising a non-integrated nucleic acid, such as a plasmid, cosmid, phagemid, or linear expression element, which comprises a sequence coding for expression of an anti-4-1BB antibody or epitope-binding fragment thereof of the invention.

Antibodies and antigen-binding fragments thereof of the invention can be produced in different cell lines, such as human cell lines, non-human mammalian cell lines, and insect cell lines, such as CHO cell lines, HEK cell lines, BHK-21 cell lines, murine cell lines (such as myeloma cell line), fibrosarcoma cell line, PER.C6 cell line, HKB-11 cell line, CAP cell line, and HuH-7 human cell line (Dumont et al., 2015, Crit Rev Biotechnol., Sep. 18, 1-13., the contents of which are incorporated herein by reference).

The antibodies of the invention are suitably separated from the culture medium by conventional immunoglobulin purification methods, such as Protein A-Sepharose, hydroxyapatite chromatography, gel electrophoresis, dialysis, or affinity chromatography.

The present invention further relates to a composition comprising, consisting of, or consisting essentially of the antibodies of the invention.

As used herein, the term "consisting essentially of" with respect to a composition means that at least one antibody of the invention as described above is the only biologically active therapeutic agent or reagent in the composition.

In one embodiment, the composition of the invention is a pharmaceutical composition and further comprises a pharmaceutically acceptable carrier.

The term "pharmaceutically acceptable carrier" refers to an excipient that does not produce adverse, allergic, or other adverse reactions when administered to animals, preferably humans, including any, and all solvents, dispersion media, coatings, antibacterial and antifungal agents, isotonic and absorption delaying agents and the like. For human administration, preparations should meet sterility, pyrogenicity, general safety and purity standards as required by regulatory agencies (e.g., FDA or EMA).

The invention further relates to a medicament comprising, consisting of, or consisting essentially of the antibodies of the invention.

In some embodiments, the glycosylation of the antibodies of the invention is modified. For example, an aglycoslated antibody can be made (i.e., the antibody lacks glycosylation). Glycosylation can be altered to, for example, increase the affinity of the antibody for an antigen or change the ADCC activity of the antibody. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for an antigen. Such an approach is described in further detail in U.S. Patent Nos. 5,714,350 and 6,350,861 by Co et al. Additionally, or alternatively, an antibody can be made that has an altered type of glycosylation, such as a hypofucosylated or an afucosylated antibody having reduced amounts of fucosyl residues or having no fucosyl residues, or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, EP 1,176,195 by Hang et al. describes a cell line with a functionally disrupted FUT8 gene, which encodes a fucosyl transferase, such that antibodies expressed in such a cell line exhibit hypofucosylation. PCT Publication WO 03/035835 by Presta describes a variant CHO cell line, Lecl3 cells, with reduced ability to attach fucose to Asn (297)-linked carbohydrates, also resulting in hypofocosylation of antibodies expressed in that host cell (see also Shields, R.L. et al., (2002), J. Biol Chem. 277: 26733-26740). PCT Publication WO 99/54342 by Umana et al., describes cell lines engineered to express glycoprotein-modifying glycosyl transferases (e.g., beta(1,4)-N-acetylglucosammyltransferase III (GnTIII)) such that antibodies expressed in the engineered cell lines exhibit increased bisecting GlcNac structures which results in increased ADCC activity of the antibodies (see also Umana et al., (1999), Nat. Biotech. 17:176-180). Eureka Therapeutics further describes genetically engineered CHO mammalian cells capable of producing antibodies with altered mammalian glycosylation patterns lacking fucosyl residues (http://www.eurekainc.com/a&boutus/ companyoverview.html). Alternatively, the human antibodies (preferably monoclonal antibodies) of the invention can be produced in yeast or filamentous fungi which are used in the production of antibodies having mammalian-like glycosylation patterns and capable of producing a glycosylation model lacking fucose (see e.g., EP1297172B1).

4-1BB (CD137/TNFRSF9) is expressed on antigen-primed T cells rather than resting T cells. Furthermore, it is known to be expressed on dendritic cells (DCs), natural killer cells (NKs), activated CD4+ and CD8+ T lymphocytes, eosinophils, natural killer T cells (NKTs) and mast cells, but myeloid-derived suppressor cells (MDSCs) do not express this molecule on their surface. Anti-4-1BB antibodies have the ability to activate cytotoxic T cells and increase the production of interferon gamma (IFN-γ). The tumor necrosis factor receptor superfamily (TNFRSF) is a protein superfamily consisting of 29 members, playing an important role in the human immune system. This family of molecules is divided into two classes: death receptors (8 members) and activation receptors. They all contain an intracellular signaling pathway activation domain and an extracellular receptor site. These receptor sites can be activated by binding to the corresponding ligands of the tumor necrosis factor superfamily (TNFSF).

The ligand-receptor (such as CD40-CD40L, CD27-CD70 or OX40-OX40L) signaling pathways of TNFSF-TNFRSF can regulate many important processes in the body, such as cell development and death, cytokines and chemokines induction. More and more evidence indicate that members of TNFRSF and TNFSF are involved in inflammations and pathologies of various diseases including cancers. In immunotherapies, an effective immune response requires two biological signals to adequately activate T cells and other immune cells. The first signal, i.e. an antigen-specific signal, is produced by the interaction of lymphocyte receptors with specific peptides bound to major histocompatibility complex (MHC) molecules on antigen-presenting cells (APCs). The second signal is a non-antigen-specific co-stimulatory signal. This type of signal is provided by the interaction between T cells and co-stimulatory molecules expressed on APCs. 4-1BB is a co-stimulatory molecule belonging to TNFRSF. It was discovered in the late 1980s when screening T cell factors, and stimulating mouse helper T cells and cytotoxic T cells with concanavalin A. Human 4-1BBL was first isolated from the activated CD4+ T lymphocyte population by direct expression cloning in 1994. 4-1BBL is mainly expressed on dendritic cells, B cells or macrophages.

The antibody of the invention acts on 4-1BB. 4-1BB, as a potent T cell-specific co-stimulatory molecule, is also expressed on many non-T cells, such as DC cells, monocytes, B cells, mast cells, NK cells and neutrophils. Activation of 4-1BB co-stimulatory signaling by anti-4-1BB agonists or 4-1BBL transfection can induce cell proliferation, cytokine expression, bactericidal activity and support T cell effector function. The binding of an agonistic antibody to 4-1BB can enhance the immune killing function of T cells, at the same time, can induce the activation of immune cells and promote the secretion of cytokines (such as IL-8).

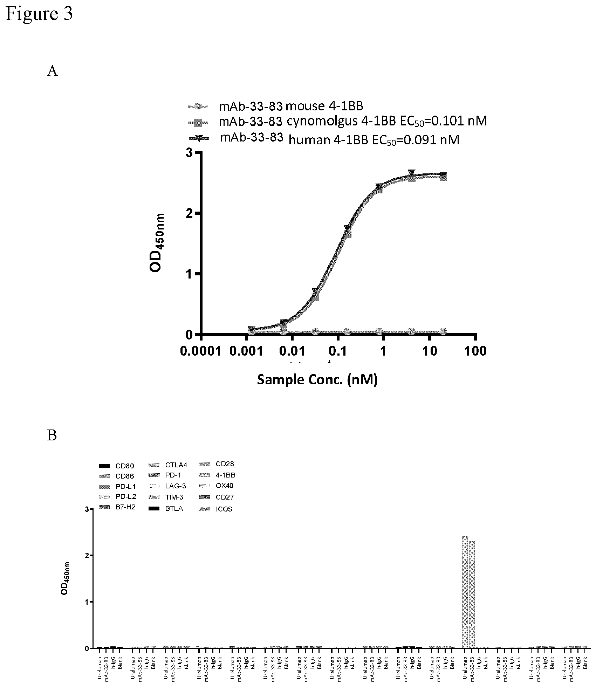

The chimeric anti-human 4-1BB monoclonal antibodies provided by the invention can bind to human 4-1BB and cynomolgus 4-1BB with a similar affinity, but do not bind to mouse 4-1BB, having species specificity. The chimeric anti-human 4-1BB monoclonal antibodies have strong binding specificity, only binding to 4-1BB, not to B7 family members, CD28 family members and CTLA-4, LAG-3, TIM-3, OX40 and CD27 proteins, as shown in Figure 3.

Where a range of values is provided, it is understood that unless the context clearly dictates otherwise, each intervening value, to the tenth of the unit of the lower limit, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference.

Embodiments of the invention will be illustrated in detail by following examples, but those skilled in the art will understand that the following examples are only for illustrating the invention, and should not be construed as limiting the scope of the invention. On the one hand, the examples provided by the invention are used to describe the preparation processes of the antibodies of the invention. The preparation processes are only to illustrate the relevant methods and is not limiting. It is well known to those skilled in the art that the invention can be modified without departing from the spirit of the invention, and such modifications also fall within the scope of the invention. On the other hand, the invention provides examples to illustrate the characteristics and advantages of the antibodies of the invention, but the invention is not limited to these characteristics and advantages.

The following experimental methods are all conventional methods unless otherwise specified. Those not indicating the specific conditions in the examples are carried out according to the conventional conditions or the conditions suggested by the manufacturer. The reagents or instruments used of which the manufacturers are not specified are all conventional products that could be commercially purchased. The various antibodies used in the following examples of the present invention are all standard antibodies commercially available.

### Examples

### Example 1 Preparation of the murine anti-human 4-1BB monoclonal antibodies

### 1.1. Animal immunizations

The 6-8-week-old female wild-type Balb/c mice (Beijing Vital River) were selected as the experimental material. After the mice acclimated to the environment for one week, the immunization began. For the first immunization, 50 µg of the recombinant human 4-1BB-Fc protein (Sino Biological Inc., catalog number 10041-H03H) and complete Freund's adjuvant (Sigma-Aldrich, catalog number F5881) were thoroughly mixed to form an emulsion and injected intraperitoneally into the mice. Two weeks later, a booster immunization was given. For the booster immunization, 25 µg of the recombinant human 4-1BB-Fc protein and incomplete Freund's adjuvant (Sigma-Aldrich, catalog number F5806) were thoroughly mixed to form an emulsion and injected intraperitoneally into the mice. The immunization was boosted with the same process every 2 weeks, a total of 3 times. On day 10 after the last immunization, blood was collected from the posterior orbital venous plexus of the mice, centrifuged to isolate serum, and the antibody titer against the recombinant human 4-1BB-His protein (Sino Biological Inc., catalog number 10041-H08H) was determined with an Enzyme Linked Immunosorbent Assay (ELISA). Mice with high titers were selected for fusion to make hybridomas. Three days before fusion, 50 µg of the recombinant human 4-1BB-His protein was injected intraperitoneally without an adjuvant. On the day of fusion, the spleen was taken aseptically, prepared into individual splenocytes suspension with DMEM medium (Gibco, catalog number 11965) for fusion.

### 1.2. Preparation of hybridomas

The myeloma cells SP2/0 (purchased from Chinese Academy of Sciences) grew to logarithmic phase, centrifuged at 1000 rpm for 5 min, the supernatant was discarded, the cells were suspended with incomplete DMEM culture medium and counted, the required number of cells were taken and were mixed together with splenocytes according to a certain ratio in a 50ml centrifuge tube, centrifuged for 5 min. The supernatant was discarded and tapping the bottom of the centrifuge tubes with your hand to loosen the precipitated cells evenly. 1 mL of PEG (Sigma, Catalog number P7181) was slowly added over 1 minute and let stand for 90 seconds. The incomplete DMEM medium was added to 50 mL to terminate fusion, let stand for 5 minutes, centrifuged at 1000 rpm for 5 minutes, and the supernatant was discarded. 20 ml of HAT medium (Sigma, catalog number: H0262-10VL) were added to disperse the fused cells gently. The fused cells were added into HAT medium at a concentration of 1×10⁵ cells per well and plated, and the cells were cultured at 37 °C in a 5% CO₂ incubator. After 7 ~ 10 days, HAT medium was replaced with HT medium (Sigma, catalog number H0137-10VL). The growth of the hybridomas was regularly observed, and the supernatant was pipetted when the hybridomas grew to more than 1/10 of the area of the bottom of the well for antibody detection. The cells of the positive clones were expanded and freezed.

### 1.3. Screening and identification of clones

An Enzyme Linked Immunosorbent Assay (ELISA) was used to screen the anti-human 4-1BB antibodies in the hybridoma culture supernatants. A 96-well high-adsorption ELISA plate was coated with 1 µg/mL of the recombinant human 4-1BB (Sino Biological Inc., catalog number 10041-H08H) at 100 µL per well using carbonate buffer solution, pH=9.6 at 4°C overnight. The plate was washed 5 times with PBST, and then blocked using PBST with 1% BSA, 300 µL/well, and incubated at 25°C for 1 hour, and then washed 5 times with PBST. The culture supernatant samples and positive serum control were added, 100 µL/well, incubated at 25°C for 1 hour, and then washed 5 times with PBST. The horseradish peroxidase-labeled anti-mouse IgG antibody (Abcam, catalog number Ab7068) diluted 1:10000 in PBST with 1% BSA was then added, 100 µL per well, and incubated at 25°C for 1 hour. The plate was washed 5 times with PBST. The colorimetric substrate TMB was added, 100 µL/well, and developed at room temperature for 10 minutes. 1M H₂SO₄ was added, 100 µL/well, to stop the development. The absorbance at 450nm was read on a microplate reader. Positive clones capable of secreting the anti-human 4-1BB selection antibodies were selected according to the magnitude of OD₄₅₀ₙₘ.

The binding of an agonistic antibody to 4-1BB can enhance the immune killing function of T cells, and at the same time, can induce the activation of immune cells and promote the secretion of cytokines (such as IL-8). The screening of the agonistic anti-human 4-1BB antibodies is based on GS-H2/4-1BB cell experiment. The hybridoma cells of the positive clones were inoculated into the peritoneal cavity of the mice, the ascites was taken after one week, purified to obtain the antibody protein. GS-H2/4-1BB cells were cultured. On the day of screening, GS-H2/4-1BB cells were digested with trypsin, the digestion was stopped with a complete medium, the cells were centrifuged at 1000rpm for 5 minutes, the supernatant was discarded, and the cells were resuspended in MEM medium (Gibco, catalog number 10370-021) containing 2% serum to a cell density of 1×10⁵ cells/mL. The cells were plated at 100 µL per well on a 96-well plate, and cultured in a 5% CO₂ incubator at 37°C for 2 hours. Biotin-labeled anti-mouse IgG antibody (Beijing Zhongshan Golden Bridge, catalog number ZB-2020) and Dynabeads Streptavidin Magnetic Beads M-280 (Invitrogen, catalog number 112-05D) were incubated at room temperature for 30 minutes, and the magnetic beads were separated by a magnet for 2-3 minutes, the magnetic beads were washed 4-5 times with PBS. After culturing for 2 hours, 50 µL of the antibodies to be tested at a final concentration of 10 µg/mL and 50 µL of the incubated magnetic beads at a final concentration of 100 µg/mL were added into each well of the cell plate, incubated in a 5% CO₂ incubator at 37°C for 24 hours. The expression of IL-8 in the supernatant of GS-H2/4-1BB cells was detected with an IL-8 detection kit (Dakewe, catalog number 1110802) according to the instructions, and the positive clones capable of secreting the agonistic anti-human 4-1BB antibodies were selected according to the magnitude at OD₄₅₀ nm.

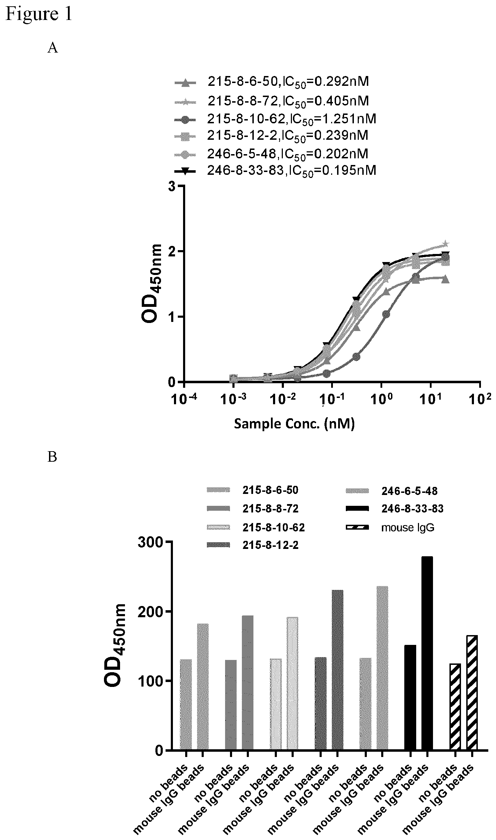

As shown in Table 1 and panel A of Figure 1, the antibodies secreted by multiple anti-human 4-1BB clones have a binding activity to 4-1BB. As shown in panel B of Figure 1, the antibodies secreted by multiple anti-human 4-1BB clones have an agonistic activity for 4-1BB.

**Table 1. Binding activity to 4-1BB of anti-human 4-1BB antibodies**

| Test sample | 4-1BB binding (EC50, nM) |
|---|---|
| 4-1BB-215-8-2-8 | 0.185 |
| 4-1BB-215-8-6-50 | 0.292 |
| 4-1BB-215-8-10-62 | 1.251 |
| 4-1BB-215-8-12-2 | 0.239 |
| 4-1BB-246-6-5-48 | 0.202 |
| 4-1BB-246-8-33-83 | 0.195 |

### 1.4. Determination of the sequences of the monoclonal antibodies

The DNA sequences of the antibodies secreted by the clones that have both the antigen-binding activity and the agonistic activity were determined. Cellular mRNAs were first extracted using the RNAprep Pure kit (Tiangen, DP419). Then the SMART 5' RACE kit (Clontech, catalog number 634849) was used to reversely transcribe the total RNAs extracted from the hybridomas into the first strand of the anti-human 4-1BB cDNAs, and the primers VHGSP (SEQ ID NO: 32) and VLGSP (SEQ ID NO: 33) were designed respectively for PCR amplification of the variable regions. The target bands obtained by PCR amplification were cloned into the linearized pRACE vector (Clontech, catalog number: 634859) in an in-fusion way, and single clones were picked up for DNA sequencing.

### Example 2 Preparation of the chimeric anti-human 4-1BB monoclonal antibodies

The sequence encoding the antibody light chain variable region of 4-1BB-246-8-33-83 clone amplified by PCR was set forth in SEQ ID NO: 7, and the sequence encoding the antibody heavy chain variable region was set forth in SEQ ID NO: 8. The complementarity determining region sequences can be obtained after excluding the framework region sequences according to the murine variable region sequences, wherein the amino acid sequences of the three complementarity determining regions LCDR1, LCDR2 and LCDR3 of the light chain are set forth in SEQ ID Nos: 1, 2 and 3 respectively, the amino acid sequences of the three complementary determining regions HCDR1, HCDR2 and HCDR3 of the heavy chain are set forth in SEQ ID Nos: 4, 5 and 6 respectively. The chimeric 4-1BB monoclonal antibodies constructed for 4-1BB-246-8-33-83 clone were named mAb-33-83, of which the light chain was obtained by linking the light chain variable region (SEQ ID NO: 7) to the light chain constant region (SEQ ID NO: 9) derived from 4-1BB-246-8-33-83 clone. The heavy chain of the mAb-33-83 antibody was obtained by linking the heavy chain variable region (SEQ ID NO: 8) to the heavy chain constant region (SEQ ID NO: 10).

The genes encoding the amino acid sequences of the light and heavy chains of the antibodies above were synthesized by Genewiz Ltd to obtain the nucleotide sequences which were then cloned into the eukaryotic cell expression vector X0GC. Then the expression vector was transfected into the ExpiCHO cell line (ExpiCHO^{™}, Catalog number A29133, invitrogen). The cells were inoculated one day before transfection, then resuspended in fresh ExpiCHO^{™} Expression Medium (ExpiCHO^{™} Expression Medium, Catalog number A29100, invitrogen), at the cell density 35 × 10⁵ cells/mL. The cell number was counted on the day of transfection and the cell density was in (70-200) ×10⁵ cells/mL range, the viability should be higher than 95%. The cells were diluted with pre-warmed ExpiCHO^{™} expression medium to the final density 60 ×10⁵ cells/mL. The plasmids and the transfection reagent ExpiFectamine^{™} CHO reagent (catalog number A29131, invitrogen) were diluted with OptiPRO^{™} medium (catalog number 12309, invitrogen) according to the transfection volume, and the final concentration of the plasmids was 0.5 µg/ml. The diluted transfection reagent was gently added to the plasmids, let it stand for 1-5 min, then the plasmid transfection reagent complex was added to the cells, the mixture was placed in the cell culture incubator, shaked at 125 rpm at 37 °C, 8% CO₂. On the next day after transfection, ExpiFectamine^{™} CHO Enhancer (Catalog number A29131, invitrogen) and ExpiCHO^{™} Feed (Catalog number A29101-02, invitrogen) were supplemented, and the cell culture incubator was adjusted to be shaked at 125 rpm, at 32 °C, 5% CO₂. Supernatants of cell cultures on day 10 after transfection were collected by centrifugation.

### Example 3 Binding activity of the chimeric anti-human 4-1BB monoclonal antibody to human 4-1BB

Enzyme-linked immunosorbent assay (ELISA) was used to determine the binding ability of the chimeric anti-human 4-1BB antibody to human 4-1BB. The detailed process is as follows: a 96-well high-adsorption ELISA plate was coated with 1 µg/mL of the recombinant human 4-1BB (Sino Biological Inc., catalog number 10041-H08H) at 100 µL per well using carbonate buffer solution, pH=9.6 at 4°C overnight. The plate was washed 5 times with PBST, and then blocked using PBST with 1% BSA, 300 µL/well, and incubated at 25°C for 1 hour, and then washed 5 times with PBST. The anti-human 4-1BB antibody samples and the control Urelumab (a patent sequence from WO2004010947) were added, 100 µL/well, incubated at 25°C for 1 hour, and then washed 5 times with PBST. The horseradish peroxidase-labeled anti-mouse IgG antibody (Abcam, catalog number Ab7068) diluted 1:10000 in PBST with 1% BSA was then added, 100 µL per well, and incubated at 25°C for 1 hour. The plate was washed 5 times with PBST. The colorimetric substrate TMB was added, 100 µL/well, and developed at room temperature for 10 minutes. 1M H₂SO₄ was added, 100 µL/well, to stop the development. The absorbance at 450nm was read on a microplate reader.

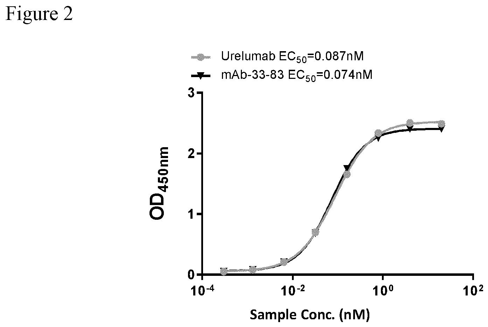

As shown in Figure 2, the chimeric anti-human 4-1BB monoclonal antibody mAb-33-83 has good binding affinity to human 4-1BB, stronger than that of the control Urelumab.

### Example 4 Species specificity and binding specificity of the chimeric anti-human 4-1BB monoclonal antibody

Species specificity of the chimeric anti-human 4-1BB mAbs was determined by an Enzyme Linked Immunosorbent Assay (ELISA). The detailed process is as follows: a 96-well high-adsorption ELISA plate was coated with 1 µg/mL of the recombinant human 4-1BB, cynomolgus 4-1BB, and mouse 4-1BB (all purchased from Sino Biological Inc.), at 100 µL per well using carbonate buffer solution, pH=9.6 at 4°C overnight. The plate was washed 5 times with PBST, and then blocked using PBST with 1% BSA, 300 µL/well, and incubated at 25°C for 1 hour, and then washed 5 times with PBST. The chimeric anti-human 4-1BB antibody samples serially diluted in PBST with 1% BSA were added, 100 µL/well, incubated at 25°C for 1 hour, and then washed 5 times with PBST. The horseradish peroxidase-labeled anti-human IgG antibody (Chemicon, catalog number AP309P) diluted 1: 10000 in PBST with 1% BSA was then added, 100 µL per well, and incubated at 25°C for 1 hour. The plate was washed 5 times with PBST. The colorimetric substrate TMB was added, 100 µL/well, and developed at room temperature for 10 minutes. 1M H₂SO₄ was added, 100 µL/well, to stop the development. The absorbance at 450nm was read on a microplate reader.

Binding specificity of the chimeric anti-human 4-1BB mAbs was determined by ELISA. The detailed process is as follows: a 96-well high-adsorption ELISA plate was coated with 1 µg/mL of the recombinant human CD80, CD86, 4-1BB, PD-L2, B7-H2, CTLA4, PD1, LAG-3, TIM-3, BTLA, CD28, 4-1BB, OX40, CD27, ICOS (CD28 was purchased from ACROBiosystems, all other proteins were purchased from Sino Biological Inc.), at 100 µL per well using carbonate buffer solution, pH=9.6 at 4°C overnight. The plate was washed 5 times with PBST, and then blocked using PBST with 1% BSA, 300 µL/well, and incubated at 25°C for 1 hour, and then washed 5 times with PBST. The chimeric anti-human 4-1BB mAb samples serially diluted in PBST with 1% BSA and the control Urelumab and the isotype control were added, 100 µL/well, incubated at 25°C for 1 hour, and then washed 5 times with PBST. The horseradish peroxidase-labeled anti-human IgG antibody (Chemicon, catalog number AP309P) diluted 1:10000 in PBST with 1% BSA was then added, 100 µL per well, and incubated at 25°C for 1 hour. The plate was washed 5 times with PBST. The colorimetric substrate TMB was added, 100 µL/well, and developed at room temperature for 10 minutes. 1M H₂SO₄ was added, 100 µL/well, to stop the development. The absorbance at 450nm was read on a microplate reader.

As shown in Figure 3 (panel A), the chimeric anti-human 4-1BB monoclonal antibody mAb-33-83 can bind to human 4-1BB and cynomolgus 4-1BB with similar affinities, but does not bind to mouse 4-1BB and has species specificity. As shown in panel B of Figure 3, the chimeric anti-human 4-1BB monoclonal antibody mAb-33-83 has very strong binding specificity, only binding to 4-1BB, not to B7 family members, CD28 family members and CTLA-4, LAG-3, TIM-3, OX40 and CD27 protein.

### Example 5 Activity of the chimeric anti-human 4-1BB monoclonal antibody of blocking the binding of 4-1BB to its receptor

The activity of the chimeric anti-human 4-1BB monoclonal antibody mAb-33-83 of blocking the binding of 4-1BB to its receptor was determined by an Enzyme Linked Immunosorbent Assay (ELISA). The detailed process is as follows: a 96-well high-adsorption ELISA plate was coated with 1 µg/mL of the recombinant human 4-1BB (Sino Biological Inc., catalog number 10041-H08H) at 100 µL per well using carbonate buffer solution, pH=9.6 at 4°C overnight. The plate was washed 5 times with PBST, and then blocked using PBST with 1% BSA, 300 µL/well, and incubated at 25°C for 1 hour, and then washed 5 times with PBST. The anti-human 4-1BB antibody samples were added, 50 µL/well, the biotin-labelled 4-1BBL (ACROBiosystems, catalog number 41L-H82F9) was added at a concentration of 200 ng/mL (a final concentration of 100 ng/mL), 50 µL/well, incubated at 25°C for 90 minutes, and then washed 5 times with PBST. The Streptavidin-HRP (BD, catalog number 554066) diluted 1:1000 in PBST with 1% BSA was then added, 100 µL per well, and incubated at 25°C for 1 hour. The plate was washed 5 times with PBST. The colorimetric substrate TMB was added, 100 µL/well, and developed at room temperature for 10 minutes. 1M H₂SO₄ was added, 100 µL/well, to stop the development. The absorbance at 450nm was read on a microplate reader.

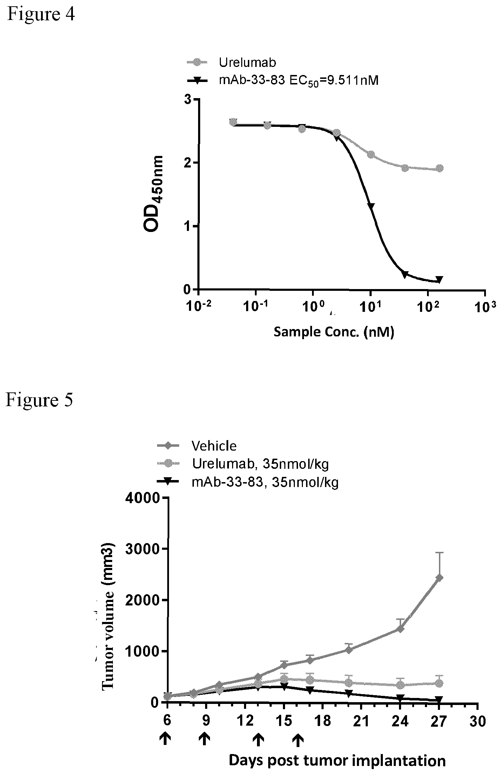

As shown in Figure 4, the chimeric anti-human 4-1BB monoclonal antibody has a 4-1BB/4-1BBL blocking activity better than that of Urelumab.

### Example 6 Anti-tumor efficacy study of the chimeric anti-human 4-1BB mAb

The 6-8-week-old female h4-1BB/h4-1BB double humanized mice (in which the murine 4-1BB and 4-1BB were knocked out, and the human 4-1BB and 4-1BB were overexpressed, Biocytogen Jiangsu Gene Biotechnology Co., Ltd) were selected as the experimental material. After the mice acclimated to the environment for one week, each mouse was subcutaneously inoculated with 5×10⁶ MC38/h4-1BB mouse colon tumor cells (MC38 cells were purchased from Shunran (Shanghai) Biotechnology Co., Ltd) on the right back. Once the tumors grow to a volume of about 100 mm³, the mice were divided into different groups according to the tumor volumes, 6 tumor-bearing mice per group. The vehicle (DPBS, GIBCO, catalog number 14190-136), the chimeric anti-human 4-1BB monoclonal antibody mAb-33-83 35 nmol/kg, and the control Urelumab 35 nmol/kg were administered respectively, twice a week for 2 consecutive weeks, via an intraperitoneal injection. The tumor volumes were measured twice a week beginning at the day of administration, and the long diameter a and the short diameter b were measured and the tumor volume calculation formula is: tumor volume (mm3) = (a × b²)/2.

As shown in Figure 5, the chimeric anti-human 4-1BB monoclonal antibody mAb-33-83 has an anti-tumor activity, significantly inhibited the growth of tumors in the homologous tumor model, with its efficacy better than that of the control Urelumab.

### Example 7 Preparation of the humanized anti-human 4-1BB monoclonal antibodies

The humanized anti-human 4-1BB mAbs were obtained according to the method described by Leung et al. (1995, Molecule Immunol 32:1413-27). The humanized templates that best match the variable region sequences of the murine antibody were selected from the Germline database, wherein the templates for the light chain variable region are hIGKV1-27*01 + hIGKJ4; the templates for the heavy chain variable region are hIGHV1-69^{∗}02 + hIGHJ6*01. The light chain CDR regions of the murine antibody were grafted into the selected humanized templates to replace the CDR regions of the human templates, obtaining the grafted, humanized antibody light chain variable region of the sequence set forth in SEQ ID NO: 11, and the grafted, humanized antibody heavy chain variable region of the sequence set forth in SEQ ID NO: 12. Sites on SEQ ID NO: 11 and SEQ ID NO: 12 were selected to carry out back mutations to obtain the light chain variable region sequences set forth in SEQ ID Nos: 13-20, and the heavy chain variable region sequences set forth in SEQ ID Nos: 21-31. The light chain variable region was linked to the light chain constant region (SEQ ID NO: 9) to obtain the corresponding full-length sequences of the light chains respectively, and the heavy chain variable region was linked to the heavy chain constant region (SEQ ID NO: 10) to obtain the corresponding full-length sequences of the heavy chains respectively. Available humanized sequences were obtained through affinity and stability screening, and the sequence information of the light and heavy chain variable regions of the obtained humanized sequences is shown in Table 2, wherein the ID columns list the abbreviations for the chimeric monoclonal antibodies and the humanized monoclonal antibodies.

**Table 2 The light and heavy chain variable region sequence information for chimeric and humanized mAbs**

| ID | VH SEQ ID NO. | VL SEQ ID NO. | ID | VH SEQ ID NO. | VL SEQ ID NO. |
|---|---|---|---|---|---|
| Chimeric mAbs | 8 | 7 | z11 | 27 | 16 |
| z0 | 12 | 11 | z12 | 28 | 16 |
| z1 | 21 | 11 | z13 | 29 | 16 |
| z2 | 21 | 13 | z14 | 30 | 16 |
| z3 | 22 | 11 | z15 | 31 | 16 |
| z4 | 22 | 13 | z16 | 26 | 15 |
| z5 | 22 | 14 | z17 | 26 | 17 |
| z6 | 22 | 15 | z18 | 26 | 14 |
| z7 | 23 | 13 | z19 | 22 | 18 |
| z8 | 24 | 13 | z20 | 22 | 19 |
| z9 | 25 | 13 | z21 | 22 | 20 |
| z10 | 26 | 16 | - | - | - |

### Example 8 Antigen-binding activity of the humanized anti-human 4-1BB monoclonal antibodies

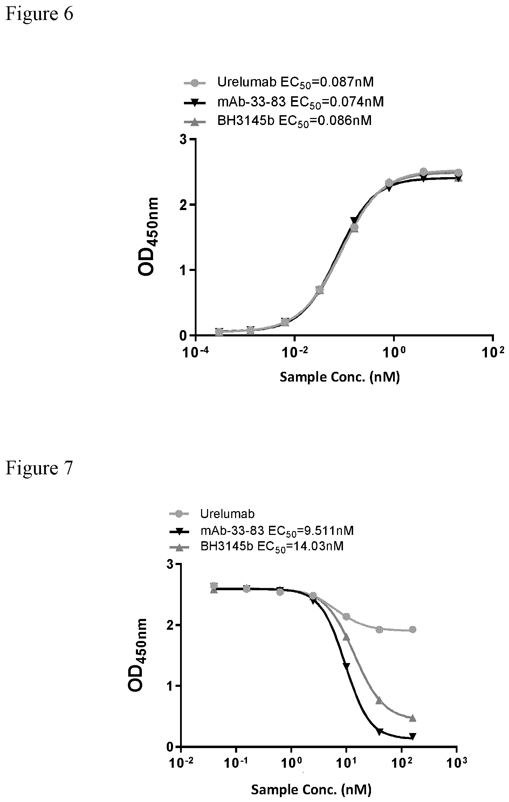

The humanized anti-4-1BB monoclonal antibody with ID z0 obtained in Example 7 was named BH3145b (sequences thereof are as follows: VH SEQ ID NO. 12; VL SEQ ID NO. 11). Detection of the antigen-binding activity of the humanized anti-human 4-1BB mAb BH3145b was performed with the steps described in Example 4. As shown in Figure 6, the humanized anti-human 4-1BB monoclonal antibody BH3145b has good binding affinity to human 4-1BB, which is comparable to that of the chimeric anti-human 4-1BB mAb mAb-33-83 and the control Urelumab.

### Example 9 Activity of the humanized anti-human 4-1BB mAb of blocking the binding of 4-1BB to its receptor

The humanized anti-4-1BB monoclonal antibody obtained in Example 7 was named BH3145b. The activity of the humanized anti-human 4-1BB mAb of blocking the binding of 4-1BB to its receptor was detected with the steps described in Example 5. As shown in Figure 7, the humanized anti-human 4-1BB mAb BH3145b has a 4-1BB/4-1BBL blocking activity less than that of the chimeric anti-human 4-1BB mAb mAb-33-83 but better than that of the control Urelumab.

Finally, it should be noted that the above examples are only used to illustrate the technical solutions of the invention, rather than to limit them. While the inventions have been described in detail with reference to the foregoing examples, those of ordinary skill in the art will recognize that numberous modifications can be made to the technical solutions described in the foregoing embodiments, or various equivalent alternatives can be made for some or all the technical features described herein without making the essence of the corresponding technical solutions depart from the scopes of various embodiments of the invention.

## Claims

1. An isolated anti-human 4-1BB antibody or antigen-binding fragment thereof, wherein the antibody or antigen-binding fragment thereof comprises three complementarity determining region LCDRs of the light chain variable region set forth in SEQ ID No. 7, and three complementarity determining region HCDRs of the heavy chain variable region set forth in SEQ ID No. 8.

2. An isolated anti-human 4-1BB antibody or antigen-binding fragment thereof, wherein the antibody or antigen-binding fragment thereof comprises a light chain variable region and/or a heavy chain variable region, wherein,
the light chain variable region comprises a LCDR1 of the amino acid sequences set forth in SEQ ID No. 1, a LCDR2 of the amino acid sequences set forth in SEQ ID No. 2, and a LCDR3 of the amino acid sequences set forth in SEQ ID No. 3; and/or
the heavy chain variable region comprises a HCDR1 of the amino acid sequences set forth in SEQ ID No. 4, a HCDR2 of the amino acid sequences set forth in SEQ ID No. 5, and a HCDR3 of the amino acid sequences set forth in SEQ ID No. 6.

3. The anti-human 4-1BB antibody or antigen-binding fragment thereof according to claim 1 or 2, wherein the anti-human 4-1BB antibody or antigen-binding fragment thereof is a chimeric antibody, a humanized antibody, or a fully human antibody.

4. The anti-human 4-1BB antibody or antigen-binding fragment thereof according to any one of claims 1-3, wherein the heavy chain constant region sequence of the antibody is the constant region sequence of one selected from the group consisting of human IgG1, IgG2, IgG3, IgG4, IgA, IgM, IgE, IgD, and/or, the light chain constant region sequence of the antibody is the constant region sequence of κ chain or λ chain; preferably, the heavy chain constant region sequence is the constant region sequence of IgG1 or IgG4, and/or the light chain constant region sequence is the constant region sequence of κ chain.

5. The anti-human 4-1BB antibody or functional fragment thereof according to any one of claims 1-4, wherein the light chain variable region has the amino acid sequence set forth in SEQ ID No. 7, and/or the heavy chain variable region has the amino acid sequence set forth in SEQ ID No. 8.

6. The anti-human 4-1BB antibody or functional fragment thereof according to any one of claims 1-4, wherein
(a) the light chain variable region has the amino acid sequence selected from those set forth in SEQ ID Nos. 13-20; and/or
the heavy chain variable region has the amino acid sequence selected from those set forth in SEQ ID Nos. 21-31;
(b) the light chain variable region has the amino acid sequence set forth in SEQ ID No. 11; and/or
the heavy chain variable region has the amino acid sequence selected from those set forth in SEQ ID Nos. 21-31;
(c) the light chain variable region has the amino acid sequence selected from those set forth in SEQ ID Nos. 13-20; and/or
the heavy chain variable region has the amino acid sequence set forth in SEQ ID No. 12.

7. The anti-human 4-1BB antibody or functional fragment thereof according to any one of claims 1-4, wherein
the light chain variable region has the amino acid sequence set forth in SEQ ID NO. 11, and/or
the heavy chain variable region has the amino acid sequence set forth in SEQ ID NO. 12.

8. The anti-human 4-1BB antibody or antigen-binding fragment thereof according to any one of claims 1-7, wherein the antigen-binding fragment is one or more selected from the group consisting of F(ab')₂, Fab', Fab, Fd, Fv, scFv, diabody, camelid antibody, CDR and antibody minimal recognition unit (dAb), preferably, the antigen-binding fragment is Fab, F(ab')₂ or scFv.

9. An isolated nucleic acid molecule selected from the group consisting of:
(1) DNA or RNA encoding the anti-human 4-1BB antibody or antigen-binding fragment thereof according to any one of claims 1-8;
(2) a nucleic acid that is completely complementary to the nucleic acid defined in (1).

10. An expression vector comprising the nucleic acid molecule according to claim 9 operably linked.

11. A host cell comprising the nucleic acid molecule according to claim 9 or the expression vector according to claim 10.

12. A composition comprising the anti-human 4-1BB antibody, antigen-binding fragment, variant or derivative thereof according to any one of claims 1-8, the nucleic acid molecule according to claim 9, the vector according to claim 10 or the host cell according to claim 11, and one or more pharmaceutically acceptable carriers, diluents or excipients.

13. A method of producing the anti-human 4-1BB antibody or antigen-binding fragment thereof according to any one of claims 1-8, comprising the steps of:
culturing the host cells according to claim 11 under culture conditions suitable for the expression of the anti-human 4-1BB antibody, antigen-binding fragment or variant thereof, and optionally, isolating and purifying the obtained products.

14. Use of the anti-human 4-1BB antibody or antigen-binding fragment thereof according to any one of claims 1-8, the nucleic acid molecule according to claim 9, the vector according to claim 10, or the host cell according to claim 11 in the manufacture of a medicament for the prevention and/or treatment of 4-1BB-mediated diseases or disorders, preferably, the diseases or disorders are tumors; more preferably, the tumors are one or more selected from the group consisting of leukemia, lymphoma, myeloma, brain tumor, head and neck squamous cell carcinoma, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell carcinoma, and melanoma.
